# EUROPEAN PATENT SPECIFICATION

(11) **EP 3 226 928 B1**
(45) Date of publication and mention of the grant of the patent: **14.10.2020**
(21) Application number: 15813177.1
(22) Date of filing: 25.11.2015
(51) Int. Cl.: A61M 1/10

(54) **MEAN ARTERIAL PRESSURE ESTIMATION**
SCHÄTZUNG DES MITTLEREN ARTERIELLEN DRUCKS
ESTIMATION D'UNE PRESSION ARTÉRIELLE MOYENNE

(30) Priority: 02.12.2014 US 201462086419 P
(43) Date of publication of application: 11.10.2017
(73) Proprietor: Heartware, Inc., Miami Lakes, FL 33014 (US)
(72) Inventor: BROWN, Michael C., Boston, MA 02115 (US); REYES, Carlos, Davie, FL 33331 (US)
(74) Representative: Zimmermann & Partner Patentanwälte mbB
(86) International application number: PCT/US2015/062617
(87) International publication number: WO 2016/089693

(56) References cited:
- WO-A1-2015/179921
- WO-A2-01/72352
- US-A- 4 588 404

## Description

### CROSS-REFERENCE TO RELATED APPLICATIONS

The present application claims the benefit of the filing date of U.S. Provisional Patent Application No. 62/086,419 filed December 2, 2014.

### BACKGROUND

Implantable blood pumps may be used to provide assistance to patients with late stage heart disease. Blood pumps operate by receiving blood from a patient's vascular system and impelling the blood back into the patient's vascular system. By adding momentum and pressure to the patient's blood flow, blood pumps may augment or replace the pumping action of the heart. For example, a blood pump may be configured as ventricular assist device or "VAD." Where a VAD is used to assist the pumping action of the left ventricle, the device typically draws blood from the left ventricle of the heart and discharges the blood into the aorta.

To provide clinically useful assistance to the heart, blood pumps impel blood at a substantial blood flow rate. For an adult human patient, a ventricular assist device may be arranged to pump blood at about 1-10 liters per minute at a differential pressure across the pump of about 10-110 mm Hg, depending on the needs of the patient. The needs of the patient may vary with age, height, and other factors.

For a patient whose vascular system is assisted by a VAD, it is desirable to regularly monitor the patient's mean arterial pressure in order to ensure that the patient does not have a high blood pressure condition. High blood pressure conditions can increase the risk of stroke or thrombus, especially in VAD users. Methods for monitoring mean arterial pressure generally require measurements to be taken by a clinician and, therefore, are inconvenient for regular monitoring, let alone continuous monitoring, of the patient's mean arterial pressure. Therefore, it is desirable to provide a blood pump controller that can monitor the mean arterial pressure of patient regularly, or even continuously, based on information gathered from the blood pump which it controls.

WO 01/72352 A2 relates to a chronic performance control system for rotodynamic blood pumps.

### SUMMARY OF THE INVENTION

One aspect of the present disclosure is directed to a method of monitoring the mean arterial pressure of a patient having a blood pump. The method may include: identifying a segment of time associated with diastole of the patient's cardiac cycle; determining a first parameter of the blood pump corresponding to the identified segment of time; and estimating the mean arterial pressure of the patient based at least in part on the first parameter. The first parameter may be indicative of a blood flow rate at the pump. The blood flow rate may be determined based on an operating current of the pump. The pump may include a stator incorporating a plurality of coils for applying a rotating magnetic field on the rotor. The blood flow rate is determined based on back electromotive force (BEMF) in one or more of the plurality of coils.

The method may further include determining an amount of pressure head exerted by the pump based at least in part on the first parameter, determining an amount of pressure loss at an outlet of the pump based at least in part on the first parameter, and determining the mean arterial pressure of the patient based at least in part on the pressure head and pressure loss amounts. The mean arterial pressure of the patient may further be determined by calculating a difference between the pressure head and pressure loss amounts. The amount of pressure head may be determined based at least in part on data that is programmed into a control circuit operatively coupled to the blood pump. For instance, the amount of pressure head may be determined based at least in part on a determined speed of a rotor of the pump. The amount of pressure loss may be determined based at least in part on data that is programmed into a control circuit operatively coupled to the blood pump.

The method may further include retrieving data at a control circuit. The segment of time associated with diastole of the patient's cardiac cycle may be identified based on the retrieved data. Alternatively, or additionally, the segment of time associated with diastole may be identified using the first parameter.

Identifying a segment of time associated with diastole may be repeatedly performed such that multiple segments are identified, each segment being associated with a different cardiac cycle of the patient. The mean arterial pressure of the patient may be independently estimated for each identified segment. In some examples, the identified segments may be associated with consecutive cardiac cycles.

Another aspect of the present disclosure is directed to a control circuit for monitoring the mean arterial pressure of a patient having a blood pump. The control circuit may be operatively coupled to the pump and may further be operative to identify a segment of time associated with diastole of the patient's cardiac cycle, to determine a first parameter of the blood pump corresponding to the identified segment of time, and to estimate the mean arterial pressure of the patient based at least in part on the first parameter.

The first parameter may be indicative of a blood flow rate at the pump. In such examples, he control circuit may be further operative to determine the blood flow rate based on an operating current of the pump.

The pump may include a stator incorporating a plurality of coils for applying a rotating magnetic field on the rotor. In such examples, the control circuit may be further operative to determine the blood flow rate based on back electromotive force (BEMF) in one or more of the plurality of coils.

In some examples, the control circuit may be operative to determine an amount of pressure head exerted by the pump based at least in part on the first parameter, to determine an amount of pressure loss at an outlet of the pump based at least in part on the first parameter, and to determine the mean arterial pressure of the patient based at least in part on the pressure head and pressure loss amounts. The control circuit may also, or otherwise, be operative to estimate the mean arterial pressure of the patient based on the difference between the pressure head and pressure loss amounts.

In some examples, the control circuit may be operatively coupled to a memory device configured to store predetermined data indicative of at least one of the amount of pressure head or the amount of pressure loss. Also, in some examples, the control circuit may retrieve data (e.g., the first parameter, other information indicative of the first parameter), and may identify a segment of time associated with diastole of the patient's cardiac cycle based on the retrieved data. The segment of time associated with diastole may be identified using peak and valley detection.

Either one, or both, of the control circuit and the remote device may be further operative to identify a segment of time associated with diastole of the patient's cardiac cycle. The first and second parameters upon which said estimation of mean arterial pressure is based may be chosen so as to correspond to the identified segment of time. In those examples in which the control device is operable to identify a segment of time associated with diastole of the patient's cardiac cycle, the control circuit may further be operable to isolate data associated with the identified segment (e.g., pump speed data, flow rate data, etc.) for transmission to the remote device. In such cases, the remote device may be operable to determine the first parameter and estimate mean arterial pressure based on the isolated data.

In some examples, the remote device may determine at least one of: an amount of pressure head exerted by the pump based at least in part on the first parameter; and an amount of pressure loss at an outlet of the pump based at least in part on the first parameter. The mean arterial pressure of the patient based at least in part on the pressure head and pressure loss amounts, such as based on the difference between the pressure head and pressure loss amounts. Determinations of pressure head and/or pressure loss made by the remote device may be based on information stored in memory of the remote device. For example, the remote device may store data indicating a relationship between pressure head exerted by the pump and flow in the pump and/or data indicating a relationship between pressure loss at an exit of the pump and flow in the pump. Such relationships may be predetermined based on characteristics of the pump.

In those cases where the remote the device determines only one of pressure head and pressure loss, the control circuit may determine the other. Data that is determined by the control circuit may be transmitted to the remote device.

In some examples, the remote device may be operable to determine an amount of pressure head exerted by the pump based at least in part on the first parameter, to determine an amount of pressure loss at an outlet of the pump based at least in part on the first parameter, and to determine the mean arterial pressure of the patient based at least in part on the pressure head and pressure loss amounts. The remote device may also, or otherwise, be operable to estimate the mean arterial pressure of the patient based on the difference between the pressure head and pressure loss amounts.

Yet a further aspect of the disclosure provides for a system for monitoring the mean arterial pressure of a patient having a blood pump. The system may include a control circuit including memory and a processor for executing instructions stored in the memory. In some cases, the control circuit may be operable to control operation of the blood pump (e.g., control pump speed). The control circuit may further be operatively coupled to the blood pump to determine a first parameter of the pump (e.g., blood flow rate, electrical current supplied, etc.). The system may also include a remote device (e.g., one or more processors and/or servers) operatively coupled to the control circuit to receive the determined first parameter, and a second parameter of the pump (e.g., pump speed) from the control circuit. The remote device may further be operative to store the first and second parameters. The remote device may estimate the mean arterial pressure of the patient based at least in part on the first and second parameters.

A further aspect of the disclosure provides for an implantable blood pump system including any of the above described control circuits (including combinations of control circuits and remote devices), and a pump. The pump may include a housing having an axis, as well as a rotor disposed within the housing, such that the rotor is rotatable around the axis.

### BRIEF DESCRIPTION OF THE DRAWINGS

FIG. 1 is an exploded perspective view of a blood pump system in accordance with an aspect of the disclosure.
FIG. 2 is a block diagram of an example control circuit of the blood pump system of FIG. 1, in accordance with an aspect of the disclosure.
FIG. 3 is a graphical plot of pressure head as a function of flow in accordance with an aspect of the disclosure.
FIGS. 4A and 4B are graphical plots of pressure loss as a function of flow in accordance with an aspect of the disclosure.
FIG. 5 is a block diagram of another example control circuit of the blood pump system of FIG. 1, in accordance with an aspect of the disclosure.
FIG. 6 is a flow diagram of a method for calculating mean arterial pressure in accordance with an aspect of the disclosure.
FIG. 7 is a graphical plot of a patient's measured mean arterial pressure compared to the estimated mean arterial pressure as a function of time in accordance with an aspect of the disclosure.

### DETAILED DESCRIPTION

The present disclosure provides for a blood pump system including a blood pump and a control circuit. The blood pump may be a centrifugal pump, such as the HVAD® Pump manufactured by HeartWare Inc. in Miami Lakes, Fla., USA. In operation, the blood pump draws blood from the left ventricle of a patient's heart and propels the blood through an outflow graft connected to the patient's ascending aorta. Although in the example of the HVAD® Pump, the blood pump is a centrifugal pump, in other examples the blood pump may be an axial flow pump, such as the MVAD® Pump, also manufactured by HeartWare Inc., or any other pump suitable for providing vascular assistance.

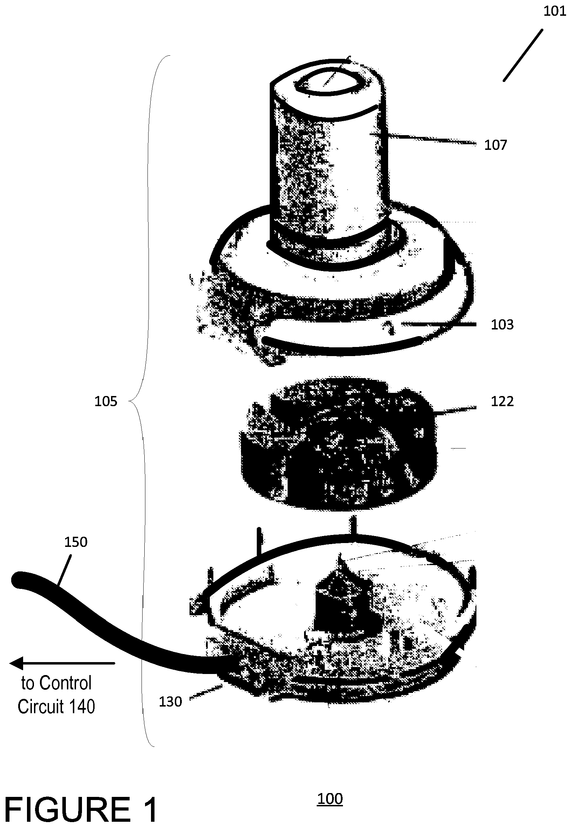

FIG. 1 depicts a blood pump system 100 in accordance with one embodiment of the invention. The blood pump system 100 according to this embodiment includes a control circuit 140 (not shown) connected via a cable feed 150 to a centrifugal blood pump 101. The blood pump 101 includes a housing 105 consisting of interlocking casings to form a closed pumping chamber 103 between them. Blood is supplied to the pump 101 through an axial inlet cannula 107 adapted for apical insertion into a heart ventricle. The cannula 107 is affixed to or may be integral with the housing 105 and is in fluid flow communication with the pumping chamber 103. Blood exits the pumping chamber 103 through an outlet 113 opposite the inlet cannula 107 in a direction substantially perpendicular to the longitudinal axis of the inlet cannula 107.

A motor rotor or pump impeller 122 is located within the pumping chamber 103. In operation, blood entering the cannula 107 from a heart ventricle passes into the pumping chamber 103 where it is engaged by the rotating impeller 122. Blood entering the pumping chamber from the cannula 107 is redirected from axial flow exiting the cannula to a radial flow within which the impeller 122 is submerged.

The housing 105 may contain an electrical feed through connector 130 for a power and control cable to supply power to the electrical motor of the pump. The cable feed 150 carrying a plurality of cables is connected to the pump through the connector 130. The cables in the feed 150 may carry electrical power and control instructions to the pump 101.

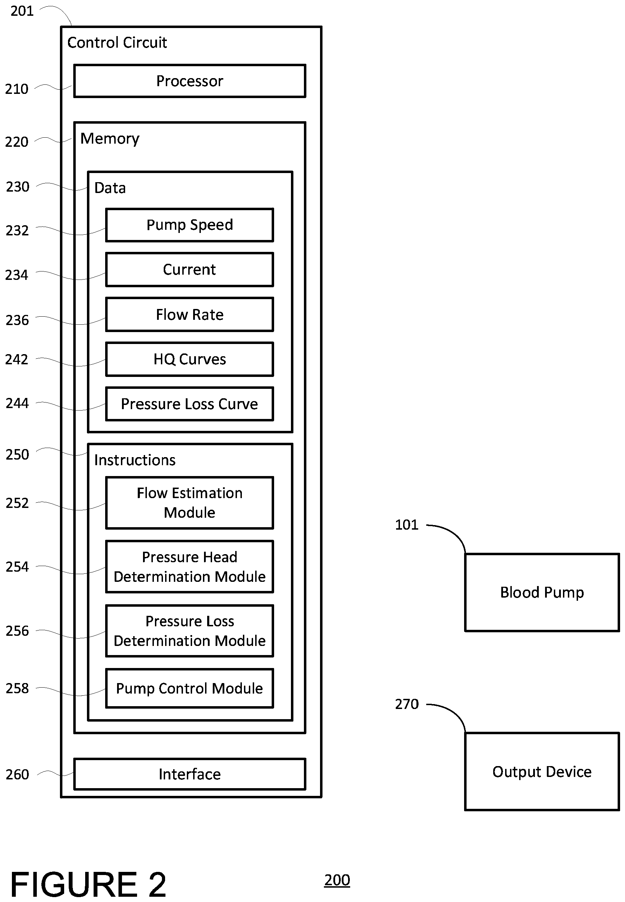

The control circuit 140 monitors and further controls operation of the pump 101. The control circuit functions may be implemented at least in part by a general-purpose processor, as shown in the example implementation of FIG. 2. As shown, an example control circuit 201 (which may be used as the control circuit 140 of FIG. 1) is implemented using a processor 210, a memory 220 and an interface 260. Memory 220 stores information accessible by processor 210, including instructions 250 that may be executed by the processor 210. The memory also includes data 230 that may be retrieved, manipulated or stored by the processor 210. The memory may be of any type capable of storing information accessible by the processor, such as a hard-drive, memory card, ROM, RAM, DVD, CD-ROM, write-capable, and read-only memories. The processor 210 may be any well-known processor, such as commercially available processors. Alternatively, the processor may be a dedicated controller such as an ASIC.

Data 230 may be retrieved, stored or modified by processor 210 in accordance with the instructions 250. The data may also be formatted in any computer-readable format such as, but not limited to, binary values, ASCII or Unicode. Moreover, the data may comprise any information sufficient to identify the relevant information, such as numbers, descriptive text, proprietary codes, pointers, references to data stored in other memories (including other network locations) or information that is used by a function to calculate the relevant data.

The control circuit includes hardware and software for controlling the various aspects of the operation of the pump. The control circuit is coupled to the pump and it is operable to collect at least some of data 230 from the pump. For example, data 230 may include pump speed data 232, indicating a speed of rotation of the pump's rotor. Pump data 230 may also include an amount of current 234 used to drive the pump. In addition, the pump data 230 may include flow rate data 236 indicative of a flow rate of blood exiting the pump when the pump is used to propel blood from the heart's left ventricle into the aorta. The flow rate data 236 may be acquired using a model for the estimation of blood flow rate. In one example, the model determines blood flow rate based in part on the acceleration of the rotor of the pump and possibly the viscosity of the patient's blood (e.g., based on hematocrit levels). Using such a model results in the estimate having a dynamic range of about 15 Hz.

In alternative embodiments, the data 230 may include further information to estimate blood flow through the pump. For example, in a centrifugal pump, the relationship between the operating current and blood flow is monotonic for the range of electrical current at which the pump may operate. Therefore, the blood flow estimate may be determined by use of a flow-to-current correlation table.

Similarly, in an axial pump, one or more flow-to-current tables may be used to estimate the blood flow rate based at least in part on a measured electrical current used to drive the pump. As explained in greater detail in commonly owned U.S. Patent Publication No. 2012/0245681, such estimates may be determined based further on the given rotor speed of the pump, a back electromotive force (BEMF) induced by the impeller on the coils of the rotor, and possibly the viscosity of the patient's blood. The estimate of blood flow may be further based at least in part on the acceleration of the rotor of the pump. Flow estimates have a dynamic range of about 15 Hz.

Additionally, different calculations and parameters may be employed to estimate a flow rate of blood. For instance, blood flow rate may be estimated algorithmically based at least in part on an operating electrical current of the pump and a predetermined hematocrit level of the blood.

In other examples, flow rate data may be collected based on other parameters indicative of flow rate. Alternatively, flow rate data may be gathered using direct measurements, such as with an ultrasonic flow meter.

Data 230 may further include one or more HQ curves 242, correlating a flow rate with a differential pressure (or differential head) exerted by the pump. In the case of the present disclosure, the HQ curves may indicate, an expected differential pressure exerted by the pump for a given flow rate of blood exiting the pump. Because the relationship between flow rate and differential pressure varies based on pump speed, different HQ curves 242 may be stored for multiple pump speeds, preferably the speeds at which the pump operates. As explained in greater detail below, the HQ curves 242 may be used to determine a differential pressure across the pump based on measured, estimated, calculated, or otherwise determined flow rate data 236.

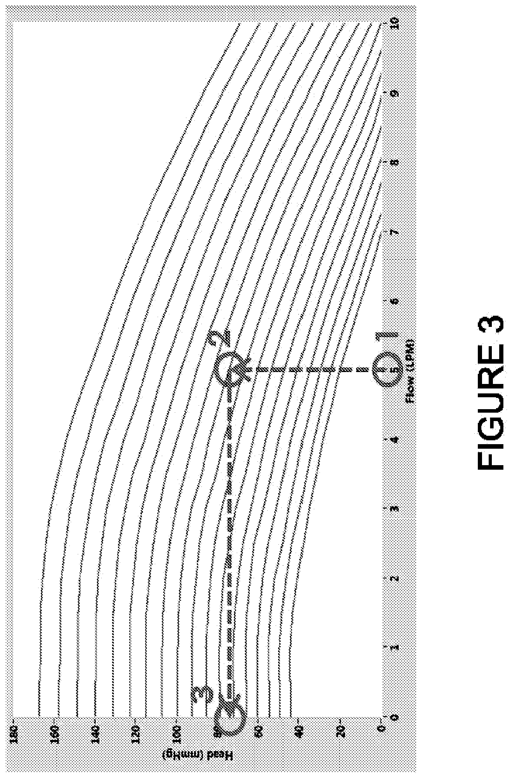

FIG. 3 shows an example set of HQ curves plotting the differential pressure as a function of flow in a given pump for a plurality of operating speeds of the pump. These curves may be predetermined during development of the pump. In the example of FIG. 3, a flow rate of 5 liters per minute (LPM) ("1") for a given known pump speed ("2") is shown to correspond to a pressure head of about 75 mmHg ("3") exerted by the pump.

Data 230 may yet further include a pressure loss curve 244 relating flow rate values with respective pressure loss values. In the case of the present disclosure, the pressure loss curve 244 may indicate an expected pressure drop between the pump's exit and a patient's aorta for a given flow rate of blood exiting the pump. The curves may be determined during development of the blood pump under test conditions. The test conditions may involve collecting several data points based on flow rate and pressure loss measurements. Since the relationship between flow rate and pressure loss varies depending on the speed of the pump, the test conditions may further involve incrementally ramping the speed of the pump, and collecting data points at each of the test speeds.

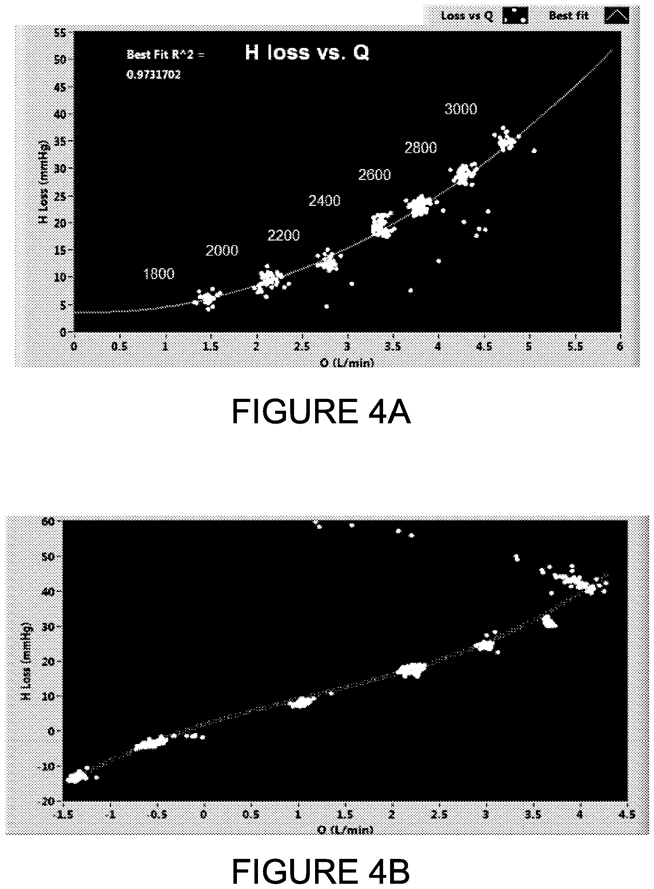

FIGS. 4A and 4B show examples of pressure loss curves that have been determined based on several data points collected under test conditions. Each of the dots in FIGS. 4A and 4B corresponds to a collected data point under the test conditions at a given pump speed (e.g., 1800 RPM, 2000 RPM... up to 3000 RPM in the example of FIG. 4A).

The collected data points may be converted into a curve by defining a polynomial best fit for the collected data points. Defining the polynomial best fit may use, for example, a second order best fit (e.g., FIG. 4A, showing a curve plotted over a range of flow values greater than zero), or a third order best fit (e.g., FIG. 4B, showing a curve plotted over a range of both positive and negative flow values). Storage of the pressure loss curve in the memory 220 may involve storage of only the polynomial best fit curve, or may further involve storage of all of the collected data points.

The instructions 250 stored in the memory 220 may include one or more instruction sets or modules for performing certain operations in accordance with the present disclosure. One such module may be a flow estimation module 252 for performing the steps required to determine a flow rate of blood through the pump. Another such module may be a pressure head determination module 254 for performing the steps required to determine pressure head exerted by the pump. A further such module may be a pressure loss determination module, pressure loss determination module 256 for performing the steps required to determine pressure loss between the pump and aorta. Yet a further module may be a pump control module 258 for controlling operation of the pump 101 (e.g., increasing or decreasing pump speed), such as in response to determination of the presence or absence of a suction condition in the pump.

The control circuit 201 may optionally include an interface 260 which connects the control circuit 201 to an output device 270. The interface 260 may be an analog interface (e.g., audio interface) or a digital interface, such as Bluetooth. TCP/IP, wi-fi, and others. Where the control circuit is implemented in an implantable structure adapted to be disposed within the body of the patient, the interface 260 may include known elements for communicating signals through the skin of the patient. The output device 270, may be a speaker, a light, a communications terminal (e.g., computer, cell phone), or any other type of device.

Although FIG. 2 functionally illustrates the processor and memory as being within the same block, it will be understood that the processor and memory may actually comprise multiple processors and memories that may or may not be stored within the same physical housing. The memory may include one or more media on which information can be stored. Preferably, the medium holding the instructions retains the instructions in non-transitory form. Some or all of the instructions and data may be stored in a location physically remote from, yet still accessible by, the processor. Similarly, the processor may actually comprise a collection of processors which may or may not operate in parallel.

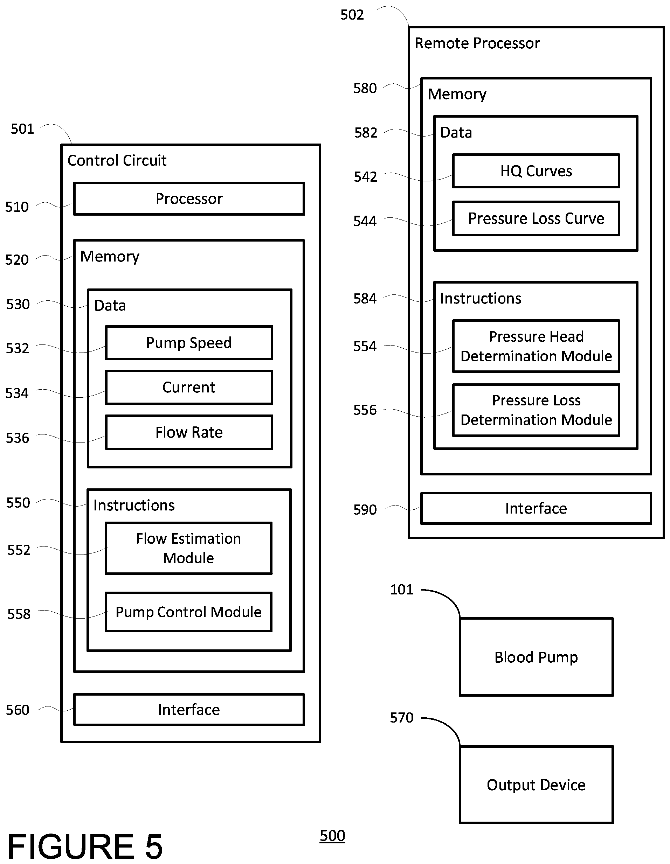

For example, FIG. 5 functionally illustrates an alternative system 500 in which a control circuit 501 having a processor 510 and memory 520 for storing data such as log pump speed data 532 and/or current data 534, as well as flow rate data 536, and for executing instructions 550 such as flow estimation 552 and pump control 558, comparable to the data and modules described in connection with FIG. 2.

The control circuit 501 also includes an interface 560 for transmitting the logged data to a remote processor 502 (e.g., computer or plurality of computers, server or plurality of servers) having an interface 590 for receiving the data. The interface 560/590 between the control circuit and remote processor may be a wired (e.g., by USB cable, Ethernet cable) or wireless (e.g., via Bluetooth, NFC, wireless network, etc.) connection.

The remote processor 502 includes its own memory 580 for storing data 582 (in addition to the logged data transmitted by the control circuit) and instructions 584. Data 580 may include the received logged data (532, 534, 536), HQ curves 542 for various pumps at various speeds, and pressure loss curves 544 for various pumps, each of which may also be comparable to the data and curves described in connection with FIG. 2. The instructions may include a pressure head determination module 554 and pressure loss determination module 556 comparable to those modules described in connection with FIG. 2.

The remote processor 550 is also operable to perform a determination of mean arterial pressure based on the logged data received from the control circuit 501.

The example systems (control circuits and/or processors) described above may be operable to estimate the mean arterial pressure of a patient using the blood pump based at least in part on the blood flow rate through the pump using the operations of the example methods described herein. It should be understood that the following operations do not have to be performed in the precise order described below. Rather, various operations can be handled in a different order or simultaneously. It should also be understood that these operations do not have to be performed all at once. For instance, some operations may be performed separately from other operations. Moreover, operations may be added or omitted.

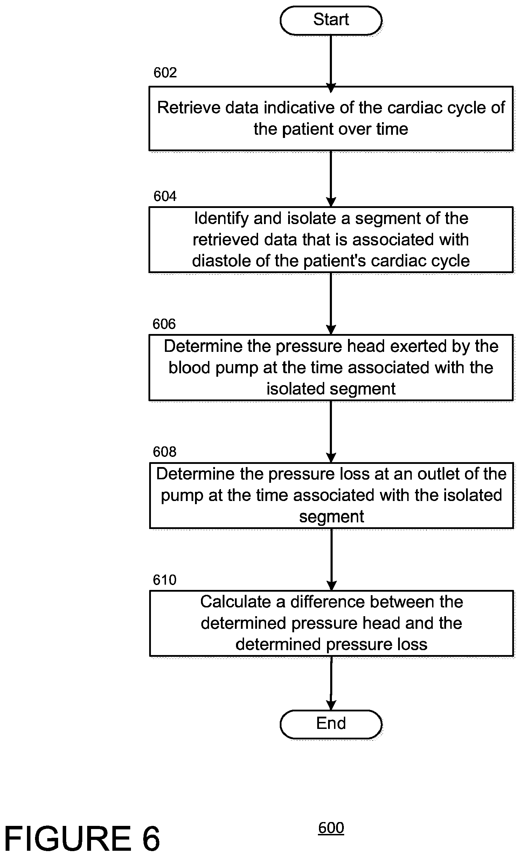

FIG. 6 is a flow diagram 600 illustrating the tasks executed by a control circuit in order to estimate the mean arterial pressure of a patient. At task 602, the control circuit retrieves data indicative of the cardiac cycle of the patient over time. For instance, the retrieved data may be flow data, and may be measured, estimated, calculated or otherwise determined. In one example, the retrieved data may be indicative of an amount of pressure at an inlet of the blood pump, which itself is estimated to be equal to the left ventricular pressure of the patient. Such data may be retrieved through direct measurement, such as by using a pressure sensor.

At task 604, the retrieved data is processed. Particularly, processing the retrieved data may involve identifying a segment of the retrieved data that is associated with diastole of the patient's cardiac cycle. It is understood that an increase in the flow rate of the flow data generally corresponds with systole, and a decrease in the flow rate generally corresponds with diastole. Therefore, a portion of the flow rate data temporally between a prior local maximum and a subsequent local minimum generally corresponds to systole. Conversely, a portion of the flow rate data temporally between a prior local minimum and a subsequent local maximum generally corresponds to diastole. In this regard, Peak and valley detection may be used to identify local maxima and minima in the flow rate data, and classify portions of the data between the identified maxima and minima as the systolic or diastolic portions of the cardiac cycle. The identified segment of task 604, which is indicative of left ventricular pressure during diastole, may be isolated for use in subsequent operations.

The processing of task 604 may be performed by the control circuit. Alternatively, in those examples that the control circuit transmits the logged flow rate data to a remote processor (such as remote processor 502 of FIG. 5), the processing may be performed either at the control circuit or at the remote processor. If the processing is performed and the flow rate data that is associated with diastole is isolated at the control circuit, then the control circuit may optionally provide only the isolated flow rate data to the remote processor, instead of sending an entire log of flow rate data.

At task 606, the pressure head exerted by the blood pump at the time associated with the isolated segment is determined. As explained above in connection with the HQ curves of FIG. 3, the pressure head may be characterized as a function of pump speed and flow rate. Thus, if the pump speed and flow rate of the pump (at the time associated with the isolated segment) are known (e.g., estimated, measured, calculated or otherwise determined), those parameters may be used to determine the pressure head.

At task 608, the pressure loss between the pump and aorta at the time associated with the isolated segment is determined. The pressure loss may be characterized as a function of flow rate. Thus, if the flow rate of the pump at the time associated with the isolated segment is known (e.g., estimated, measured, calculated or otherwise determined), the flow rate may be used to determine the pressure loss.

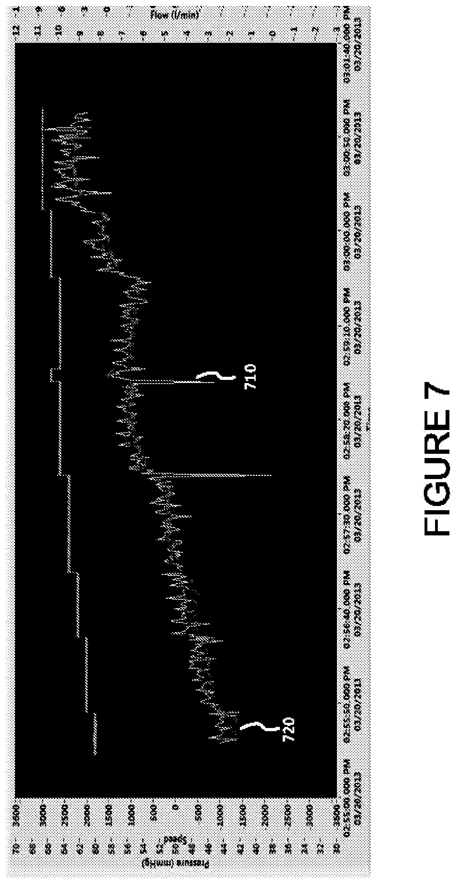

At task 610, a difference between the determined pressure head (task 606) and the determined pressure loss (task 608) is calculated. The calculated difference has been found to accurately represent the diastolic portion of the mean arterial pressure of the patient, which itself is an estimation of mean arterial pressure. To illustrate the accuracy of this calculation, FIG. 7 depicts estimated mean arterial pressure values that were determined using the method of flow diagram 600 over a span of time (curve 710), as well as actual diastolic portions of mean arterial pressure measurements taken over that same time (curve 720). As shown from FIG. 7, the measured and estimated mean arterial pressure values are substantially equal to one another. The above described tasks provide a method for determining an estimate of the mean arterial pressure of a patient, particularly during diastole of a single cardiac cycle. If the control circuit is operable to perform each of the above described tasks, the method may be repeatedly implemented by the control circuit to repeatedly provide an estimated mean arterial pressure during diastole of multiple cardiac cycles of the patient. Alternatively, log data of multiple cardiac cycles may first be collected at task 602, and then tasks 604-610 may be repeatedly implemented for each of (or a selection of) the multiple cardiac cycles represented by the data collected in task 602. Thus, the above described method allows for regular or even continuous monitoring of the patient's mean arterial pressure, even of consecutive cardiac cycles, without the inconveniences associated with conventional blood pressure measurements. The above described method also allows for collection of necessary data from a blood pump in order that the patient's mean arterial pressure may be determined at a remote location and/or a later time.

In the above examples, the estimation of mean arterial pressure is based on the assumption that the differential pressure across the pump minus the pressure loss at the outlet of the pump is equal to the aortic pressure. This assumption is in turn predicated on the estimation of left ventricular pressure to be 0 mmHg during the diastolic portion of the cardiac cycle. In other embodiments, preload on the left ventricle may be estimated so as to achieve a more accurate estimation of left ventricular pressure. In turn, such estimation would yield a more accurate estimation of mean arterial pressure.

Although the invention herein has been described with reference to particular embodiments, it is to be understood that these embodiments are merely illustrative of the principles and applications of the present invention. It is therefore to be understood that modifications may be made to the illustrative embodiments and that other arrangements may be devised without departing from the scope of the present invention as defined by the appended claims.

## Claims

1. A method (600) of monitoring the mean arterial pressure of a patient having a blood pump (101), comprising
identifying a segment of time associated with diastole of the patient's cardiac cycle (604),
determining a first parameter of the blood pump corresponding to the identified segment of time,
and estimating the mean arterial pressure of the patient based at least in part on the first parameter, further comprising
determining an amount of pressure head exerted by the pump based at least in part on the first parameter (606),
determining an amount of pressure loss at an outlet of the pump based at least in part on the first parameter (608),
determining the mean arterial pressure of the patient based at least in part on the pressure head and pressure loss amounts (610).

2. A method as recited in claim 1, wherein the first parameter is indicative of a blood flow rate at the pump, specifically wherein the blood flow rate is determined based on an operating current of the pump.

3. A method as recited in claim 1, wherein the pump includes a stator incorporating a plurality of coils for applying a rotating magnetic field on the rotor, and wherein the blood flow rate is determined based on back electromotive force (BEMF) in one or more of the plurality of coils.

4. A method as recited in claim 1, wherein the amount of pressure head is determined based at least in part on data that is programmed into a control circuit (140) operatively coupled to the blood pump, or wherein the amount of pressure head is determined based at least in part on a determined speed of a rotor of the pump, or wherein the amount of pressure loss is determined based at least in part on data that is programmed into a control circuit (140) operatively coupled to the blood pump.

5. A method as recited in claim 1, further comprising retrieving data at a control circuit (140), wherein the segment of time associated with diastole of the patient's cardiac cycle is identified based on the retrieved data, specifically wherein the segment of time associated with diastole is identified using the first parameter.

6. A method as recited in claim 1, wherein identifying a segment of time associated with diastole is repeatedly performed such that multiple segments are identified, each segment associated with a different cardiac cycle of the patient, and wherein the mean arterial pressure of the patient is independently estimated for each identified segment, specifically wherein the identified segments are associated with consecutive cardiac cycles.

7. A control circuit for monitoring the mean arterial pressure of a patient having a blood pump (101), the control circuit (140), when in use, operatively coupled to the pump and further operative to:
identify a segment of time associated with diastole of the patient's cardiac cycle,
determine a first parameter of the blood pump corresponding to the identified segment of time,
and estimate the mean arterial pressure of the patient based at least in part in the first parameter, further operative
to retrieve from a memory (220) in communication with the control circuit an amount of pressure head exerted by the pump based at least in part on the first parameter and an amount of pressure loss at an outlet (113) of the pump based at least in part on the first parameter;
determine the mean arterial pressure of the patient based at least in part on the pressure head and pressure loss amounts.

8. A control circuit as recited in claim 7, wherein the first parameter is indicative of a blood flow rate at the pump, specifically wherein the control circuit is further operative to determine the blood flow rate based on an operating current of the pump.

9. A control circuit as recited in claim 7, wherein the pump includes a stator incorporating a plurality of coils for applying a rotating magnetic field on the rotor, and wherein the control circuit is further operative to determine the blood flow rate based on back electromotive force (BEMF) in one or more of the plurality of coils.

10. A control circuit as recited in claim 7, wherein the control circuit is further operative to retrieve data, and to identify the segment of time associated with diastole of the patient's cardiac cycle based on the retrieved data.

11. A control circuit as recited in claim 10, wherein the control circuit is further operative to identify the segment of time associated with diastole using peak and valley detection, or wherein the retrieved data is or is indicative of the first parameter.

12. An implantable blood pump system comprising a control circuit as recited in claim 7 and a pump, the pump including a housing (105) having an axis, and a rotor disposed within the housing, the rotor being rotatable around the axis.

13. A system as recited in claim 12, further including a remote device operatively coupled to the control circuit to receive the determined first parameter and a second parameter of the pump from the control circuit and to estimate the mean arterial pressure of the patient based at least in part on the received first and second parameters;

14. A system as recited in claim 13, wherein the first parameter is indicative of a flow rate of blood at the pump, and the second parameter is indicative of a speed of the pump.

15. A system as recited in claim 14, wherein the remote device is further operative to determine at least one of: an amount of pressure head exerted by the pump based at least in part on the first parameter; and an amount of pressure loss at an outlet of the pump based at least in part on the first parameter, and wherein the remote device is operative to determine the mean arterial pressure of the patient based at least in part on the pressure head and pressure loss amounts.

## Patentansprüche

1. Verfahren (600) zum Überwachen des mittleren arteriellen Drucks eines Patienten, das eine Blutpumpe (101) aufweist, umfassend
Identifizieren eines mit einer Diastole des Herzzyklus (604) des Patienten verknüpften Zeitsegments,
Bestimmen eines ersten Parameters der Blutpumpe, der dem identifizierten Zeitsegment entspricht, und
Schätzen des mittleren arteriellen Drucks des Patienten, wenigstens teilweise basierend auf dem ersten Parameter, ferner umfassend
Bestimmen einer durch die Pumpe ausgeübten Druckhöhenmenge, wenigstens teilweise basierend auf dem ersten Parameter (606),
Bestimmen einer Druckverlustmenge an einem Auslass der Pumpe, wenigstens teilweise basierend auf dem ersten Parameter (608),
Bestimmen des mittleren arteriellen Drucks des Patienten, wenigstens teilweise basierend auf der Druckhöhen- und der Druckverlustmenge (610).

2. Verfahren nach Anspruch 1, wobei der erste Parameter eine Blutflussrate an der Pumpe anzeigt, insbesondere wobei die Blutflussrate basierend auf einem Betriebsstrom der Pumpe bestimmt wird.

3. Verfahren nach Anspruch 1, wobei die Pumpe einen Stator beinhaltet, der mehrere Spulen zum Anlegen eines rotierenden Magnetfelds an den Rotor enthält, und wobei die Blutflussrate basierend auf der gegenelektromotorischen Kraft *(back electromotive force -* BEMF) in einer oder mehreren der mehreren Spulen bestimmt wird.

4. Verfahren nach Anspruch 1, wobei die Druckhöhenmenge wenigstens teilweise basierend auf Daten bestimmt wird, die in einer Steuerschaltung (140) programmiert sind, die mit der Blutpumpe wirkgekoppelt ist, oder wobei die Druckhöhenmenge wenigstens teilweise basierend auf einer bestimmten Drehzahl eines Rotors der Pumpe bestimmt wird, oder wobei die Druckverlustmenge wenigstens teilweise basierend auf Daten bestimmt wird, die in einer Steuerschaltung (140) programmiert sind, die mit der Blutpumpe wirkgekoppelt ist.

5. Verfahren nach Anspruch 1, ferner umfassend ein Abrufen von Daten an einer Steuerschaltung (140), wobei das mit der Diastole des Herzzyklus des Patienten verknüpfte Zeitsegment basierend auf den abgerufenen Daten identifiziert wird, insbesondere wobei das mit der Diastole verknüpfte Zeitsegment unter Verwendung des ersten Parameters identifiziert wird.

6. Verfahren nach Anspruch 1, wobei das Identifizieren eines mit der Diastole verknüpften Zeitsegments derart wiederholt durchgeführt wird, dass mehrere Segmente identifiziert werden, wobei jedes Segment mit einem anderen Herzzyklus des Patienten verknüpft ist, und wobei der mittlere arterielle Druck des Patienten für jedes identifizierte Segment unabhängig geschätzt wird, insbesondere wobei die identifizierten Segmente mit aufeinanderfolgenden Herzzyklen verknüpft sind.

7. Steuerschaltung zum Überwachen des mittleren arteriellen Drucks eines Patienten, die eine Blutpumpe (101) aufweist, wobei die Steuerschaltung (140), wenn sie verwendet wird, mit der Pumpe wirkgekoppelt ist und ferner für Folgendes betriebsfähig ist:
Identifizieren eines mit der Diastole des Herzzyklus des Patienten verknüpften Zeitsegments,
Bestimmen eines ersten Parameters der Blutpumpe, der dem identifizierten Zeitsegment entspricht, und
Schätzen des mittleren arteriellen Drucks des Patienten, wenigstens teilweise basierend auf dem ersten Parameter, ferner für Folgendes betriebsfähig:
Abrufen, aus einem Speicher (220), der mit der Steuerschaltung kommuniziert, einer durch die Pumpe ausgeübten Druckhöhenmenge, wenigstens teilweise basierend auf dem ersten Parameter, und einer Druckverlustmenge an einem Auslass (113) der Pumpe, wenigstens teilweise basierend auf dem ersten Parameter;
Bestimmen des mittleren arteriellen Drucks des Patienten, wenigstens teilweise basierend auf der Druckhöhen- und der Druckverlustmenge.

8. Steuerschaltung nach Anspruch 7, wobei der erste Parameter eine Blutflussrate an der Pumpe anzeigt, insbesondere wobei die Steuerschaltung ferner betriebsfähig ist, um die Blutflussrate basierend auf einem Betriebsstrom der Pumpe zu bestimmen.

9. Steuerschaltung nach Anspruch 7, wobei die Pumpe einen Stator beinhaltet, der mehrere Spulen zum Anlegen eines rotierenden Magnetfelds an den Rotor enthält, und wobei die Steuerschaltung ferner betriebsfähig ist, um die Blutflussrate basierend auf der gegenelektromotorischen Kraft (BEMF) in einer oder mehreren der mehreren Spulen zu bestimmen.

10. Steuerschaltung nach Anspruch 7, wobei die Steuerschaltung ferner betriebsfähig ist, um Daten abzurufen, und um das mit der Diastole des Herzzyklus des Patienten verknüpfte Zeitsegment basierend auf den abgerufenen Daten zu identifizieren.

11. Steuerschaltung nach Anspruch 10, wobei die Steuerschaltung ferner betriebsfähig ist, um das mit der Diastole des Herzzyklus des Patienten verknüpfte Zeitsegment unter Verwendung von Spitzen- und Talerfassung zu identifizieren, oder wobei die abgerufenen Daten der erste Parameter sind oder diesen anzeigen.

12. Implantierbares Blutpumpensystem, umfassend eine Steuerschaltung nach Anspruch 7 und eine Pumpe, wobei die Pumpe ein Gehäuse (105), das eine Achse aufweist, und einen in dem Gehäuse angeordneten Rotor beinhaltet, wobei der Rotor um die Achse rotierbar ist.

13. System nach Anspruch 12, ferner umfassend eine Fernvorrichtung, die mit der Steuerschaltung wirkgekoppelt ist, um den bestimmten ersten Parameter und einen zweiten Parameter der Pumpe aus der Steuerschaltung zu empfangen und den mittleren arteriellen Druck des Patienten wenigstens teilweise basierend auf dem empfangenen ersten und zweiten Parameter zu schätzen;

14. System nach Anspruch 13, wobei der erste Parameter eine Blutflussrate an der Pumpe anzeigt und der zweite Parameter eine Drehzahl der Pumpe anzeigt.

15. System nach Anspruch 14, wobei die Fernvorrichtung ferner betriebsfähig ist, um Folgendes zu bestimmen: eine durch die Pumpe ausgeübte Druckhöhenmenge, wenigstens teilweise basierend auf dem ersten Parameter; und/oder eine Druckverlustmenge an einem Auslass der Pumpe, wenigstens teilweise basierend auf dem ersten Parameter, und wobei die Fernvorrichtung betriebsfähig ist, um den mittleren arteriellen Druck des Patienten wenigstens teilweise basierend auf der Druckhöhen- und Druckverlustmenge zu bestimmen.

## Revendications

1. Procédé (600) de monitorage de la pression artérielle moyenne d'un patient présentant une pompe à sang (101), comprenant
l'identification d'un segment de temps associé à la diastole du cycle cardiaque du patient (604),
la détermination d'un premier paramètre de la pompe à sang correspondant au segment de temps identifié,
et l'estimation de la pression artérielle moyenne du patient sur la base au moins en partie du premier paramètre, comprenant en outre
la détermination d'une quantité de charge de pression exercée par la pompe sur la base au moins en partie du premier paramètre (606),
la détermination d'une quantité de perte de pression à une sortie de la pompe sur la base au moins en partie du premier paramètre (608),
la détermination de la pression artérielle moyenne du patient sur la base au moins en partie des quantités de charge de pression et de perte de pression (610).

2. Procédé selon la revendication 1, dans lequel le premier paramètre indique un débit sanguin au niveau de la pompe, en particulier dans lequel le débit sanguin est déterminé sur la base d'un courant de fonctionnement de la pompe.

3. Procédé selon la revendication 1, dans lequel la pompe comporte un stator incorporant une pluralité de bobines visant à appliquer un champ magnétique rotatif sur le rotor, et dans lequel le débit sanguin est déterminé sur la base de la force contre-électromotrice (FCEM) dans une ou plusieurs de la pluralité de bobines.

4. Procédé selon la revendication 1, dans lequel la quantité de charge de pression est déterminée sur la base au moins en partie de données qui sont programmées dans un circuit de commande (140) fonctionnellement couplé à la pompe à sang, ou dans lequel la quantité de charge de pression est déterminée sur la base au moins en partie d'une vitesse déterminée d'un rotor de la pompe, ou dans lequel la quantité de perte de pression est déterminée sur la base au moins en partie de données qui sont programmées dans un circuit de commande (140) fonctionnellement couplé à la pompe à sang.

5. Procédé selon la revendication 1, comprenant en outre la récupération de données au niveau d'un circuit de commande (140), dans lequel le segment de temps associé à la diastole du cycle cardiaque du patient est identifié sur la base des données récupérées, en particulier dans lequel le segment de temps associé à la diastole est identifié à l'aide du premier paramètre.

6. Procédé selon la revendication 1, dans lequel l'identification d'un segment de temps associé à la diastole est effectuée de manière répétée de telle sorte que de multiples segments sont identifiés, chaque segment étant associé à un cycle cardiaque différent du patient, et dans lequel la pression artérielle moyenne du patient est indépendamment estimée pour chaque segment identifié, en particulier dans lequel les segments identifiés sont associés à des cycles cardiaques consécutifs.

7. Circuit de commande destiné au monitorage de la pression artérielle moyenne d'un patient présentant une pompe à sang (101), le circuit de commande (140) lors de l'utilisation étant couplé de manière fonctionnelle à la pompe et en outre fonctionnant pour :
identifier un segment de temps associé à la diastole du cycle cardiaque du patient,
déterminer un premier paramètre de la pompe à sang correspondant au segment de temps identifié,
et estimer la pression artérielle moyenne du patient sur la base au moins en partie du premier paramètre, fonctionnant en outre pour
récupérer à partir d'une mémoire (220) en communication avec le circuit de commande une quantité de charge de pression exercée par la pompe sur la base au moins en partie du premier paramètre et une quantité de perte de pression à une sortie (113) de la pompe sur la base au moins en partie du premier paramètre ;
déterminer la pression artérielle moyenne du patient sur la base au moins en partie des quantités de charge de pression et de perte de pression.

8. Circuit de commande selon la revendication 7, dans lequel le premier paramètre indique un débit sanguin au niveau de la pompe, en particulier dans lequel le circuit de commande est en outre fonctionnel pour déterminer le débit sanguin sur la base d'un courant de fonctionnement de la pompe.

9. Circuit de commande selon la revendication 7, dans lequel la pompe comporte un stator incorporant une pluralité de bobines visant à appliquer un champ magnétique rotatif sur le rotor, et dans lequel le circuit de commande est en outre fonctionnel pour déterminer le débit sanguin sur la base de la force contre-électromotrice (FCEM) dans une ou plusieurs de la pluralité de bobines.

10. Circuit de commande selon la revendication 7, dans lequel le circuit de commande est en outre fonctionnel pour récupérer des données et pour identifier le segment de temps associé à la diastole du cycle cardiaque du patient sur la base des données extraites.

11. Circuit de commande selon la revendication 10, dans lequel le circuit de commande est en outre fonctionnel pour identifier le segment de temps associé à la diastole à l'aide de la détection de crêtes et de creux, ou dans lequel les données récupérées sont ou indiquent le premier paramètre.

12. Système de pompe à sang implantable comprenant un circuit de commande selon la revendication 7 et une pompe, la pompe comportant un boîtier (105) présentant un axe ainsi qu'un rotor disposé à l'intérieur du boîtier, le rotor pouvant pivoter autour de l'axe.

13. Système selon la revendication 12, comportant en outre un dispositif distant couplé de manière opérationnelle au circuit de commande pour recevoir le premier paramètre déterminé et un second paramètre de la pompe à partir du circuit de commande et pour estimer la pression artérielle moyenne du patient sur la base au moins en partie des premier et second paramètres reçus ;

14. Système selon la revendication 13, dans lequel le premier paramètre indique un débit sanguin au niveau de la pompe, et le second paramètre indique une vitesse de la pompe.

15. Système selon la revendication 14, dans lequel le dispositif distant fonctionne en outre pour déterminer : une quantité de charge de pression exercée par la pompe sur la base au moins en partie du premier paramètre ; et/ou une quantité de perte de pression à une sortie de la pompe sur la base au moins en partie du premier paramètre, et dans lequel le dispositif distant fonctionne pour déterminer la pression artérielle moyenne du patient sur la base au moins en partie des quantités de charge de pression et de perte de pression.
